# EUROPEAN PATENT APPLICATION

(11) **EP 4 681 655 A1**
(43) Date of publication of application: **21.01.2026**
(21) Application number: 24773898.2
(22) Date of filing: 04.03.2024
(51) Int. Cl.: A61B 17/00, A61B 34/30

(54) **SURGICAL TOOL AND SURGICAL ROBOT SYSTEM**

(30) Priority: 17.03.2023 CN 202310263441; 17.03.2023 CN 202310263440; 17.03.2023 CN 202310263439
(71) Applicant: Beijing Surgerii Robotics Company Limited, Haidian District, 100 192 Beijing, (CN)
(72) Inventor: XU, Kai, Beijing 100192 (CN); REN, Yitang, Beijing 100192 (CN); ZHAO, Jiangran, Beijing 100192 (CN)
(74) Representative: karo IP
(86) International application number: PCT/CN2024/079852
(87) International publication number: WO 2024/193330

(57) **Abstract**

The present disclosure relates to the field of medical instruments. Disclosed are a surgical tool and a surgical robot system. The surgical tool comprises an arm body, an end instrument, at least one sliding block and at least one driving wire. The end instrument is arranged at a distal end of the arm body. The at least one sliding block comprises a connecting interface provided on the sliding block, and the connecting interface is configured to couple with an external device and receive driving from the external device. A first end of the at least one driving wire is fixedly connected to the sliding block, and the sliding block is configured to push and/or pull the driving wire under the received driving. A driving device is externally arranged and/or a transmission mechanism for transmitting driving is externally arranged, so that the miniaturization and light weight of the surgical tool are achieved.

## Description

### CROSS REFERENCES TO RELATED APPLICATIONS

The present application claims the benefit of priority to the Chinese patent application filed on March 17, 2023 with the application number 202310263441X and titled "Integrated Driving device and Surgical Robot System", the Chinese patent application filed on March 17, 2023 with the application number 2023102634405 and titled "Compact Surgical Tool and Surgical Robot System", the Chinese patent application filed on March 17, 2023 with application number 2023102634392 and titled "Connecting adapter, Connecting assembly and Surgical Robot System Capable of Transmitting Linear Motion", the entire contents of which are incorporated herein by reference in their entirety.

### TECHNICAL FIELD

The present disclosure relates to the field of medical instruments, and particularly to a surgical tool and a surgical robot system.

### BACKGROUND

Minimally invasive surgery has many characteristics, such as small trauma area and quick recovery, and has been increasingly widely used in clinical surgery. In a surgical robot system, a surgical tool is typically provided on a robotic arm. A servo motor is provided on the robotic arm, and a connecting adapter is provided between the robotic arm and the surgical tool. Torque and loads are transmitted in real time through the connecting adapter. A transmission mechanism is provided on the surgical tool to convert the rotary motion input by the motor into linear motion, thereby controlling the surgical tool, and realizing surgical operations at different parts by controlling the surgical effector at the end of the surgical tool.

During the surgery, since part of the robotic arm will directly contact the surgical tool, it is easy to be contaminated during the operation due to its proximity to the surgical part. Furthermore, the driving module of the robotic arm part generally cannot be sterilized by conventional methods such as steam, autoclaving or chemicals. The non-sterile robotic arm is isolated from the sterile surgical tool in operating environment typically with a sterile protective sleeve extending from the connecting adapter.

The existing connecting adapter is mainly used for transmitting rotational torque. In the process of frequent rotation of the surgical tool, it is easy to cause the redundant sterile protective sleeve to be entangled or drooped, and even may be involved, thus obstructing and restricting the motion of the surgical tool. Moreover, the surgical tool needs to be provided with a transmission mechanism, which will also cause the size of the surgical tool to be large, which is not conducive to carrying and assembling. Existing driving devices are mainly used to provide rotational torque and have limited application scenarios.

### SUMMARY OF THE INVENTION

In some embodiments, the present disclosure provides a surgical tool comprising:
an arm body;
an end instrument arranged at a distal end of the arm body;
at least one sliding block comprising a connecting interface provided on the sliding block, the connecting interface configured to couple with an external device and receive driving from the external device; and
at least one driving wire configured for driving the arm body and/or the end instrument, wherein a first end of the driving wire is fixedly connected with the sliding block, and the sliding block is configured for pushing and/or pulling the driving wire under the received driving.

In some embodiments, the present disclosure provides a surgical robot system comprising:
at least one robotic arm comprising at least one driving device, wherein the at least one driving device comprising at least one driving interface, the at least one driving device configured to drive the at least one driving interface to move;
at least one surgical tool as described in any embodiment of the present disclosure; and
at least one connecting adapter configured for detachable connection with the robotic arm and the surgical tool, the connecting adapter comprising at least one first interface for coupling with a connecting interface of the surgical tool and at least one second interface for coupling with the driving interface.

### BRIEF DESCRIPTION OF THE DRAWINGS

In order to explain the technical solutions in the embodiments of the present disclosure more clearly, the accompanying drawings used in the description of the embodiments of the present disclosure will be briefly introduced below. The accompanying drawings in the following description only show some of the embodiments of the present disclosure, and for those of ordinary skill in the art, other embodiments would also have been obtained from the contents of the embodiments of the present disclosure and these accompanying drawings without involving any inventive effort.
FIG. 1 shows a structural schematic diagram of a surgical tool according to some embodiments of the present disclosure;
FIG. 2 shows a structural schematic diagram of a proximal interior of a surgical tool according to some embodiments of the present disclosure;
FIG. 3 shows a structural schematic diagram of a proximal portion of a surgical tool according to some embodiments of the present disclosure;
FIG. 4 shows a structural schematic diagram of a proximal portion of a surgical tool coupled with an external device according to some embodiments of the present disclosure;
FIG. 5 shows a structural schematic diagram of the distribution of sliding blocks and driving wires according to some embodiments of the present disclosure;
FIG. 6 shows another structural schematic diagram of the distribution of sliding blocks and driving wires according to some embodiments of the present disclosure;
FIG. 7 shows a structural block diagram of a surgical robot system according to some embodiments of the present disclosure;
FIG. 8 shows an exploded structural schematic diagram of a connecting interface, a transmission member, and a driving interface according to some embodiments of the present disclosure;
FIG. 9 shows a front view of a connecting adapter in an expanded state according to some embodiments of the present disclosure;
FIG. 10 shows a structural schematic diagram of a connecting adapter in a folded state according to some embodiments of the present disclosure;
FIG. 11 shows a structural schematic diagram of a first side of the transmission member according to some embodiments of the present disclosure;
FIG. 12 shows a structural schematic diagram of a second side of the transmission member according to some embodiments of the present disclosure;
FIG. 13 shows a simplified front view of a connecting adapter in an expanded state according to some embodiments of the present disclosure;
FIG. 14 shows a structural schematic diagram of a connecting adapter assembling with a surgical tool according to some embodiments of the present disclosure;
FIG. 15 shows a rear view of a connecting adapter in an expanded state according to some embodiments of the present disclosure;
FIG. 16 shows a structural schematic diagram of the interior of a driving device according to some embodiments of the present disclosure;
FIG. 17 shows a first-perspective structural schematic diagram of a driving device according to some embodiments of the present disclosure;
FIG. 18 shows a second-perspective structural schematic diagram of a driving device according to some embodiments of the present disclosure;
FIG. 19 shows a structural schematic diagram of a transmission mechanism mating with a surgical tool according to some embodiments of the present disclosure;
FIG. 20 shows a structural schematic diagram of an integral rotation mechanism according to some embodiments of the present disclosure;
FIG. 21 shows a structural schematic diagram of a linear mechanism mating with a driving device according to some embodiments of the present disclosure;
FIG. 22 shows a structural schematic diagram of a linear mechanism according to some embodiments of the present disclosure;
FIG. 23 shows a partial structural schematic diagram of a driving device according to some embodiments of the present disclosure.

### DETAILED DESCRIPTION

To make the solved technical problems, used technical solutions, and achieved technical effects of the present disclosure more clearly, the technical solutions of the embodiments of the present disclosure will be further described in detail below with reference to the accompanying drawings. Obviously, the described embodiments are only exemplary embodiments, but not all of embodiments, of the present disclosure.

In the description of the present disclosure, it should be noted that, orientational or positional relationships indicated by the terms "center", "upper", "lower", "left", "right", "vertical", "horizontal", "inner", "outer" and the like are the orientational or positional relationships shown based on the accompanying drawings, and are only for ease of describing the present disclosure and simplifying the description, rather than indicating or implying that the apparatus or element referred to must have a specific orientation or be constructed and operated in a specific orientation, and therefore cannot be construed as limiting the present disclosure. In addition, the terms "first" and "second" are used for descriptive purposes only, and cannot be understood as indicating or implying relative importance. In the description of the present disclosure, it should be noted that, unless otherwise specified and defined, the term "mount", "connected", and "connect", or "couple" should be comprehended in a broad sense. For example, the term may be a fixed connection or a detachable connection; or may be a mechanical connection or an electrical connection; may be a direct connection or an indirect connection via an intermediate medium; or may be internal communication between two elements. For those of ordinary skill in the art, specific meanings of the foregoing terms in the present disclosure may be understood based on specific situations. In the present disclosure, an end close to an operator (e.g., a surgeon) is defined as a proximal end, a proximal portion, or a rear end, a rear portion, and an end opposite to the proximal end, the proximal portion, or the rear end, the rear portion is defined as a distal end, a distal portion, or a front end, a front portion. Alternatively, an end close to an object to be operated (e.g., a patient) is defined as a distal end, a distal portion, or a front end, a front portion, and an end opposite to the distal end, the distal portion, or the front end, the front portion is defined as a proximal end, a proximal portion, or a rear end, a rear portion. It may be understood by those skilled in the art that the embodiments of the present disclosure may be used for a medical instrument or a surgical robot, and may also be used for other non-medical apparatus.

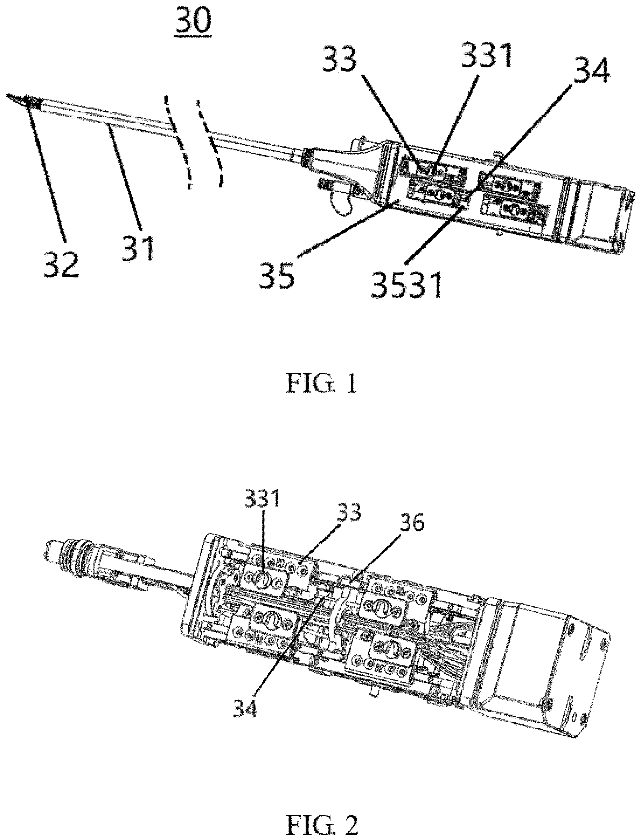

FIG. 1 shows a structural schematic diagram of a surgical tool 30 according to some embodiments of the present disclosure. As shown in FIG. 1, the surgical tool 30 may include an arm body 31, an end instrument 32 arranged at a distal end of the arm body 31, at least one sliding block 33, and at least one driving wire 34. Among them, the end instrument 32 may include an end effector or an endoscope. The end effector may include, for example, a dissector, a grasper, scissors, an electrocautery hook, a bipolar grasper, curved monopolar scissors, a needle holder, a clip applier, a drainage tube, or a suction device, and the like. The endoscope may include, for example, at least one imaging unit and an illumination unit, and the like.

The at least one sliding block 33 may include a connecting interface 331 provided on the sliding block. The connecting interface 331 is configured to couple with an external device and receive driving from the external device. For example, the external device may include a driving interface. The connecting interface 331 is directly or indirectly coupled with the driving interface to receive the driving of the external device. The at least one driving wire 34 is configured to drive the arm body and/or the end instrument. A first end of the at least one driving wire 34 may be fixedly connected with the sliding block 33. The sliding block 33 is configured to push and/or pull the driving wire 34 under the received driving. It is to be understood that the driving may include, but is not limited to, linear driving, curved driving, and the like.

In some embodiments, the driving wire 34 may include a flexible cord. The sliding block 33 may pull the flexible cord to move under the received driving. In some embodiments, the driving wire 34 may include an elastic rod, a superelastic wire, or the like, to achieve push and pull motion under the driving of the sliding block 33. The arm body and the end instrument may employ a variety of suitable structures. They may also connect to the driving wire in various suitable manners, and may perform various operations under the driving of the driving wire. For example, the arm body may include a rigid arm body or a flexible arm body. A driving device is externally arranged and/or a transmission mechanism for transmitting driving is externally arranged, so that the miniaturization and light weight of the surgical tool may be achieved.

In some embodiments, as shown in FIG. 1, the surgical tool 30 may further include a housing 35 for accommodating the at least one sliding block 33. The housing 35 includes at least one housing window (e.g., the housing window 3531), in which the connecting interface 331 of the at least one sliding block 33 is located. For example, the housing 35 may be located at a proximal end of the surgical tool 30, the at least one sliding block 33 being slidably provided within the housing 35, and the connecting interface 331 of the sliding block 33 projecting in the housing window of the housing 35. It is to be understood that a coupling structure, for example, a coupling groove, a side section, and the like, is provided on the connecting interface 331. The structure of the connecting interface on each sliding block may be the same or different. For example, the connecting interface may have an irregular cylindrical shape (for example, including a side section), or the connecting interface may have a cylindrical shape or a circular table shape, or the like. Assembly may be facilitated by setting connecting interfaces to have different structures.

FIG. 2 shows a structural schematic diagram of a proximal interior of a surgical tool 30 according to some embodiments of the present disclosure. As shown in FIGs. 1 and 2, in some embodiments, at least one guiding mechanism, for example, a sliding rail 36, may be provided within the housing 35. The at least one sliding block 33 is provided on the at least one sliding rail 36. The motion of the sliding block 33 is more stable and reliable through the sliding rail 36. It is to be understood that the guiding mechanism may also be a guiding rod. The at least one sliding block 33 may be slidably provided through at least one guiding rod. The guiding mechanism may also be a sliding groove or the like. The above are only examples, the disclosure is not limited thereto, and the guiding mechanism may also be any other structures enabling the guiding function.

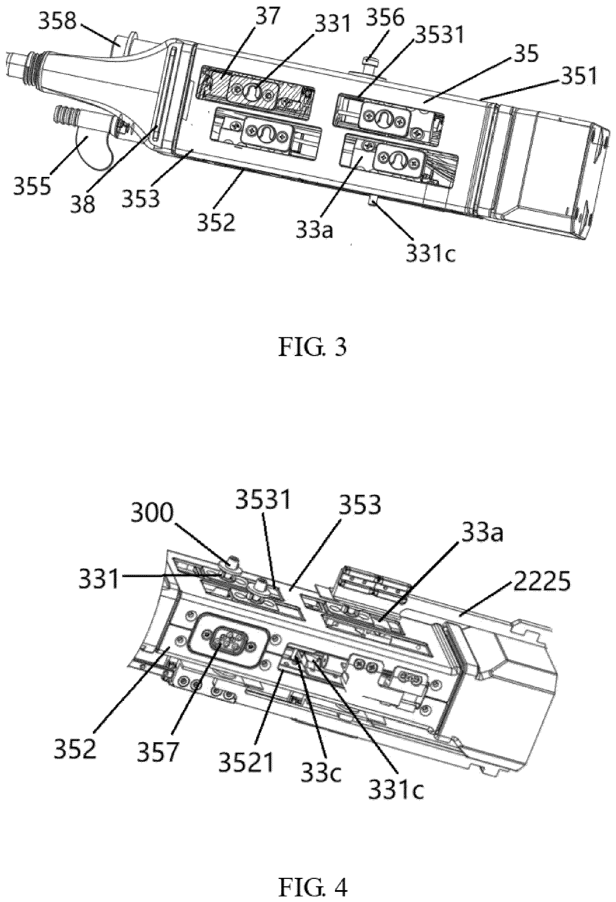

In some embodiments, FIG. 3 shows a structural schematic diagram of a proximal portion of a surgical tool 30 according to some embodiments of the present disclosure, and FIG. 4 shows a structural schematic diagram of a proximal portion of a surgical tool 30 coupled with an external device (e.g., the driving device 21) according to some embodiments of the present disclosure. For clarity, only a first deformable membrane 37 (shown in shadow) of one of the housing windows in FIG. 3 is shown, the first deformable membranes of the other housing windows in the figure are not shown. As shown in FIG. 3, the surgical tool 30 may further include at least one first deformable membrane 37. The first deformable membrane 37 is sealingly provided on the housing 35 for covering at least one housing window (e.g. housing windows 3531, 3521). It is to be understood that the circumference of the first deformable membrane 37 may be sealingly connected (e.g. welded, bonded, etc.) to the housing 35. By covering at least one housing window, an effective barrier, for example, a sterile barrier against bacteria, is formed between the first side (e.g. inner side) and the second side (e.g. outer side) of the housing 35. The connecting interface 331 is sealingly connected with the first deformable membrane 37 and is configured to receive driving through the deformation of the at least one first deformable membrane 37. For example, the connecting interface 331 may be integrally molded, welded, or bonded with the first deformable membrane 37, so that there is no gap between the connecting locations of the connecting interface 331 and the first deformable membrane 37 to sealingly isolate the contaminated area and the sterile area.

Those skilled in the art will understand that the sealing arrangement or sealing connection in the present disclosure means that the connecting location is sealed, for example, by welding, adhesion, or the like, to form a blocking barrier, for example, a sterile barrier for blocking bacteria, a dust barrier for blocking dust, or the like. The connecting interface 331 of the at least one sliding block 33 is used for transmission between the connecting interface 331 and the driving interface of the external device through deformation of the at least one first deformable membrane 37. For example, the connecting interface 331 may be configured to move under the driving of the driving interface of the external device. As shown in FIG. 4, the driving interface of the external device may be connected with the transmission member 300 through the connecting rod 23. The connecting interface 331 (or 331c) is driven to move by the transmission member 300. The first deformable membrane 37 is deformable, allowing the driving interface to drive the connecting interface 331 to move, to enable transferring of the driving (e.g. linear driving) of the driving interface to the connecting interface 331. The sliding block 33 is driven, through the connecting interface 331, to move to enable pushing and/or pulling of the driving wire 34. As a result, the structure is simple and operation is easy.

It may be understood by those skilled in the art that the motion may include various forms of motion, for example, movement parallel to the housing window plane, movement perpendicular to the housing window plane, or a combination of both the motions, and the like. The motion may include, for example, linear motion, curved motion, and the like. In some embodiments of the present disclosure, linear motion is described as an example, but this does not constitute any limitation to the present disclosure.

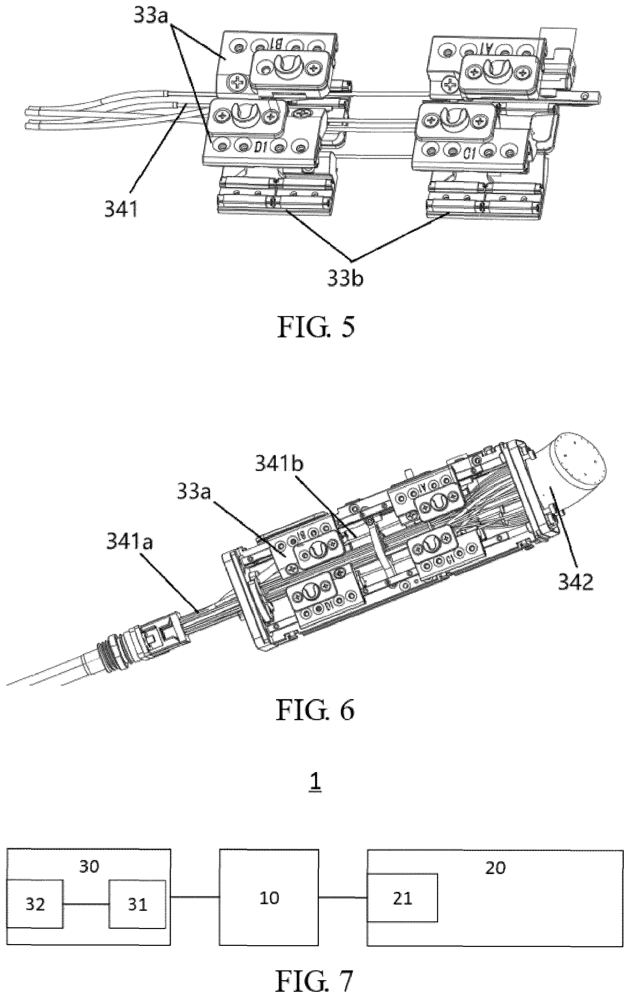

In some embodiments, as shown in FIGs. 3 and 4, the at least one housing window may include at least one first housing window 3531 located at a first side of the housing 35 and at least one second housing window (not shown) located at a second side of the housing 35. As shown in FIG. 3, the housing 35 may have a cubic structure. The housing 35 may include an upper side plate 351, a lower side plate 352, a left side plate 353, and a right side plate (not shown in FIG. 3, on the side opposite to the left side plate 353). For example, the first housing window 3531 and the second housing window (not shown) are provided on the left side plate 353 and the right side plate (not shown), respectively. As shown in FIG. 3, four first housing windows 3531 are provided on the left side plate 353. The at least one sliding block 33 may include at least one first sliding block 33a and at least one second sliding block 33b (as shown in FIG. 5). The connecting interface 331 of the at least one first sliding block 33a is located in the at least one first housing window 3531, and the connecting interface 331 of the at least one second sliding block 33b is located in the at least one second housing window.

In some embodiments, as shown in FIGs. 3 and 4, the at least one housing window may further include a third housing window 3521 located at a third side of the housing (e.g., the lower side plate 352), and the at least one sliding block 33 further includes a third sliding block 33c. The connecting interface 331c of the third sliding block 33c is located in the third housing window 3521. It may be understood by those skilled in the art that the left side, the right side, the upper side, and the lower side referred to herein are for the convenience of representing the relative positional relationship, and should be interpreted broadly, and other namings, for example, the top side, the bottom side, the front side, the rear side, and the like may also be adopted.

The at least one first housing window 3531 may include one or more first housing windows, for example, a set of first housing windows, located on the left side plate 353. The at least one second housing window may include one or more second housing windows, for example, a set of second housing windows, located on the right side plate. The at least one third housing window 3521 may include one or more third housing windows, for example, a set of third housing windows, located on the lower side plate 352. In some embodiments, taking the first sliding block 33a as an example, the connecting interfaces 331 of the plurality of first sliding blocks 33a may be located in the same first housing window 3531, or the plurality of connecting interfaces 331 of one first sliding block 33a may be located in the same first housing window 3531.

In some embodiments, the first housing window 3531 and the at least one first sliding block 33a may be arranged mirror symmetrically with respect to the second housing window 3531 and the at least one second sliding block 33b. It is to be understood that the set of first housing windows 3531 and the set of second housing windows may each include a plurality of spaced-apart housing windows, and the set of third housing windows 3521 may include one housing window. As shown in FIG. 3, each sliding block may include one connecting interface 331. As shown in FIG. 3, the numbers of the first housing windows 3531 and the first sliding blocks 33a are four, respectively, and the connecting interface 331 of each first sliding block 33a is located in the corresponding first housing window 3531, respectively. The numbers of the second housing windows and the second sliding blocks 33b are four, respectively, and the connecting interface 331 of each second sliding block 33b is located in the corresponding second housing window, respectively. The four first housing windows 3531 and the connecting interfaces of the first sliding blocks 33a are arranged mirror-symmetrically with respect to the four second housing windows and the connecting interfaces of the second sliding blocks 33b in the direction of linear movement, for example, the direction of the length of the housing 35 or the surgical tool 30. In this way, the cooperative driving of the connecting interfaces 331 located within the first housing windows 3531 and the second housing windows may be facilitated. The above numbers are merely examples. It is to be understood that the numbers of the first housing windows 3531 and the second housing windows 3531 may also be one, two, five, and the like. The number of connecting interfaces 331 located in the same housing window may also be two, three, or the like. The numbers of housing windows and connecting interfaces 331 may be adjusted according to the number of interfaces on the external device to be driven.

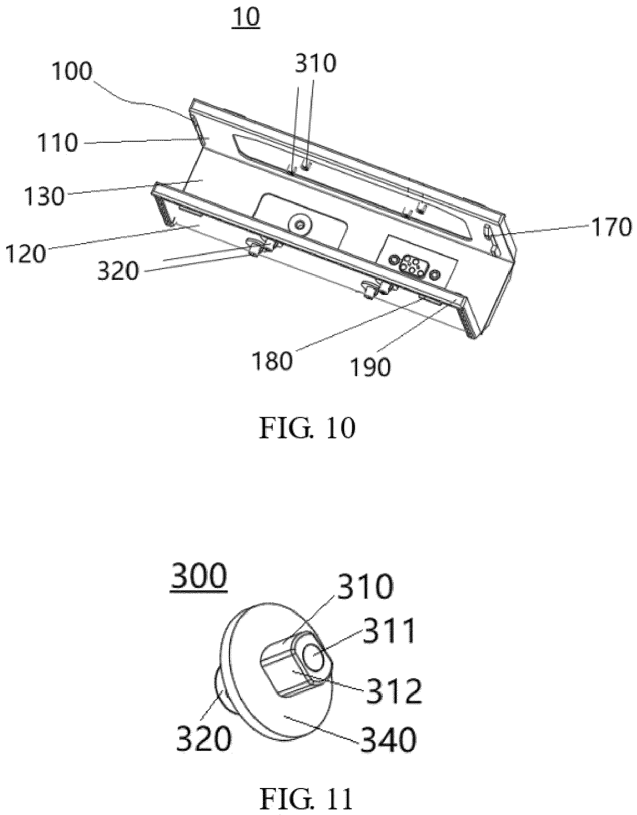

In some embodiments, as shown in FIG. 3, the surgical tool 30 may further include at least one stop structure 38 provided on the first side and/or the second side. For example, at least one stop structure 38 is provided at the outer side of the left side plate 353 and/or the right side plate. It is to be understood that the inner sides of the left side plate and the right side plate refer to the sides facing each other when the left side plate 353 and the right side plate are arranged opposite each other. The outer sides of the left side plate and the right side plate refer to the sides facing away from each other when the left side plate and the right side plate are arranged opposite each other. As shown in FIG. 3, the stop structure 38 may be a groove, and the inner side of the external device (for example, the connecting adapter 10) is provided with a stop protrusion (for example, the protrusion 170 shown in FIG. 10) mating with the stop structure 38. The stop structure engages with the stop protrusion to restrict movement of the surgical tool 30 in a direction of linear movement, for example, the direction of the length of the left side plate 353 or the right side plate. It is to be understood by those skilled in the art that the stop structure shown in FIG. 3 is only an exemplary embodiment, and other structures for example, protrusions, magnetic attraction structures, and the like, may be adopted.

In some embodiments, as shown in FIG. 3, the surgical tool 30 may further include a balancing valve 355 provided on the housing 35. The balancing valve 355 may be used to maintain balanced internal and external air pressures during sterilization of the surgical tool. During actual use, the balancing valve may be removed.

In some embodiments, as shown in FIG. 3, the surgical tool 30 may further include a handle 356 provided on the housing 35. For example, the handle 356 may be located at the outer side of the upper side plate 351 of the housing 35 for handling by an operator, so as to facilitate mounting of the surgical tool to or detachment of the surgical tool from an external device.

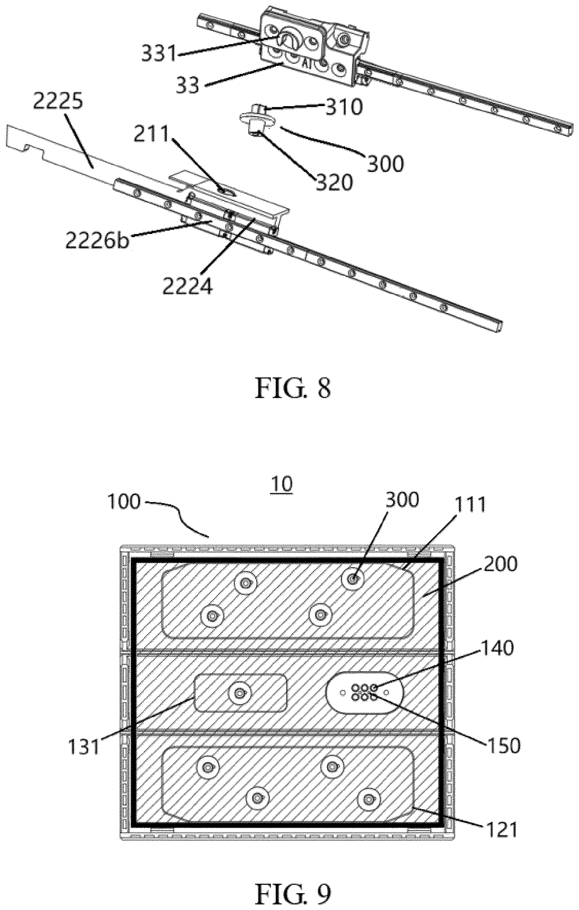

In some embodiments, as shown in FIG. 4, the surgical tool 30 may further include at least one communication interface 357 provided on the housing 35. For example, the at least one communication interface 357 may form a communication connection between the interior and the exterior of the lower side plate 352 of the surgical tool. For example, the outer side of the housing 35 of the surgical tool 30 is connected with an external device (e.g. the connecting adapter 10), and the at least one communication interface 357 may be configured to communicatively connect with the external device to form a communication connection between the inner side of the housing 35 of the surgical tool 30 and the external device (e.g. the connecting adapter 10 and the robotic arm 20) at the outer side of the housing 35 of the surgical tool 30. As shown in FIG. 4, a plurality of communication interfaces 357 spaced apart may be provided on the lower side plate 352. A plurality of communication contacts (for example, the communication contacts 140 shown in FIG. 9) are provided on the external device accordingly. After the mounting of the external device is completed, the plurality of communication interfaces 357 are conducted with the communication contacts to form a communication connection between the surgical tool 30 and the external device. It is to be understood by those skilled in the art that an assembler may determine whether the surgical tool 30 and the external device are mounted in place by determining whether the communication contacts form a communication connection with the communication interface 357. It is also possible to read parameter information on the surgical tool 30 or the external device or to write parameter information to the surgical tool 30 or the external device through a communication connection formed between the communication contacts and the communication interface 357.

In some embodiments, as shown in FIG. 3, the surgical tool 30 may further include an electrode interface 358 or an optical fiber interface (not shown) provided on the housing 35. For example, the electrode interface 358 may provide an electrically conductive path to an electrical energy tool, for example, a monopolor or bipolar electrocoagulation tool, an electrotomy tool, or the like. The fiber optic interface may provide access for endoscopic imaging or illumination when the end instrument of the surgical tool is an endoscope.

FIG. 5 shows a structural schematic diagram of the distribution of sliding blocks and driving wires according to some embodiments of the present disclosure. FIG. 6 shows another structural schematic diagram of the distribution of sliding blocks and driving wires according to some embodiments of the present disclosure. In some embodiments, as shown in FIG. 5, the at least one driving wire 34 may include at least one arm body driving wire 341. The first end of the at least one arm body driving wire 341 is fixedly connected with the first sliding block 33a and/or the second sliding block 33b, and the second end of the arm body driving wire 341 is directly or indirectly connected to the arm body 31. For example, the first end of the arm body driving wire 341 may be a proximal end of the driving wire (e.g., the right side shown in FIG. 5), and the second end of the arm body driving wire 341 may be a distal end of the driving wire (e.g., the left side shown in FIG. 5).

In some embodiments, as shown in FIG. 6, the arm body driving wire 341 may include a first arm body driving wire 341a and a second arm body driving wire 341b. The distal end of the first arm body driving wire 341a may be connected with the arm body 31. The proximal end of the first arm body driving wire 341a is indirectly connected with the proximal end of the second arm body driving wire 341b (e.g., indirectly connected by a proximal driving continuum 342). The distal end of the second arm body driving wire 341b is fixedly connected with the first sliding block 33a and/or the second sliding block 33b. The second arm body driving wire 341b is driven to push and/or pull by the first sliding block 33a and the second sliding block 33b. The push and/or pull of the second arm body driving wire 341b drives the first arm body driving wire 341a to push and/or pull. For example, the second arm body driving wire 341b drives the proximal driving continuum 342 to bend to push and/or pull the first arm body driving wire 341a by the proximal driving continuum 342, so as to achieve bending of the arm body 31.

In some embodiments, the at least one driving wire may include at least one effector driving wire (not shown). The distal end of the at least one effector driving wire is connected with an end effector. The proximal end of the effector driving wire is fixedly connected with the third sliding block 33c. The effector driving wire is driven to push and/or pull by the third sliding block 33c to achieve opening and closing of the end effector, so as to complete corresponding surgical operations, for example, clamping, grasping, cutting, and the like.

In some embodiments, at least a portion of the arm body 31 is deformable or bendable, one or more of the at least one arm body driving wire 341 are provided throughout the arm body 31, and the push and/or pull motion of the arm body driving wire 341 is used to drive the deformable portion to deform or drive the bendable portion to bend. It is to be understood that the deformable or bendable structure of the arm body 31 may include, but is not limited to, articulated arm bodies (for example, snake bone structure arm bodies), flexible arm bodies (for example, flexible tubes), and continuum arm bodies.

In some embodiments, the first deformable membrane 37 includes a sheet membrane adapted to the shape of the housing 35. The sheet membrane is sealingly provided on the inner or outer surface of the housing 35 to cover the at least one housing window. For example, the housing 35 has a cubic shape. Each side plate (e.g., the upper side plate 351, the lower side plate 352, the left side plate 353, and the right side plate) has a rectangular shape. The first deformable film 37 may be a rectangular sheet membrane adapted to each side plate. The first deformable membrane is sealingly connected with the circumferential edge of each side plate so that the first deformable membrane 37 is affixed to the surface of each side plate to cover a plurality of housing windows on each side plate.

In some embodiments, the first deformable membrane 37 includes at least one sheet membrane corresponding to the at least one housing window, respectively, and the at least one sheet membrane is sealingly connected with the housing 35 to cover the at least one housing window (e.g., the first housing window 3531, the second housing window, the third housing window 3521), respectively. It is to be understood that the at least one housing window may be rectangular, polygonal, circular, other regular or irregular shapes, or a combination of multiple shapes, or the like. The first deformable film 37 may include sheet membranes, the number of which is consistent with the number of housing windows. The shapes of the sheet membranes may match the shapes of individual housing windows. Each sheet membrane is sealingly connected with the circumferential edge of each housing window, respectively, to cover each housing window.

FIG. 7 shows a structural block diagram of a surgical robot system 1 according to some embodiments of the present disclosure. FIG. 8 shows an exploded structural schematic diagram of a connecting interface 331, a transmission member 300, and a driving interface 211 according to some embodiments of the present disclosure. As shown in FIG. 7, the surgical robot system 1 may include at least one robotic arm 20, at least one connecting adapter 10, and at least one surgical tool 30 in any one of the embodiments of the present disclosure. In some embodiments, the robotic arm 20 may include a driving device 21 located at the end. The connecting adapter 10 is detachably connected with the end of the robotic arm 20 or the driving device 21. The proximal portion of the surgical tool 30 is detachably connected with the connecting adapter 10. The robotic arm 20 is connected with the surgical tool 30 by the connecting adapter 10. The driving (for example, linear motion) of the driving device 21 is transferred to the surgical tool 30 via the connecting adapter 10 to drive the arm body 31 of the surgical tool 30 to bend and/or the end effector 32 to open and close.

As shown in FIGs. 7 and 8, the at least one robotic arm 20 may include at least one driving device 21. The at least one driving device 21 may include at least one driving interface 211. The at least one driving device 21 is connected with the driving interface 211 via a connecting rod 23 to drive the at least one driving interface 211 to move. It is to be understood that the at least one robotic arm 20 may include a plurality of robotic arms, each including one driving device 21, respectively. Alternatively, the at least one robotic arm 20 may include one robotic arm, the distal end of which may include a plurality of driving devices 21 independent of each other. At least one connecting adapter 10 is configured for detachable connection with the robotic arm 20 and the surgical tool 30. The connecting adapter 10 includes at least one transmission member 300. The transmission member 300 may include a first interface 310 for coupling with the connecting interface 331 of the surgical tool 30 and a second interface 320 for coupling with the driving interface 211.

FIG. 9 shows a front view of a connecting adapter 10 in an expanded state according to some embodiments of the present disclosure. FIG. 10 shows a structural schematic diagram of the connecting adapter 10 in a folded state according to some embodiments of the present disclosure. For convenience of illustration, the shadow indicating the second deformable membrane 200 is omitted in FIG. 10, and only the transmission member 300 is retained. In some embodiments, as shown in FIGS. 9 and 10, the connecting adapter 10 may include an adapter substrate 100, at least one second deformable membrane 200 (shown in shadow), and at least one transmission member 300. The adapter substrate 100 includes at least one transmission window (e.g. transmission windows 111, 121, and 131). The adapter substrate 100 is detachably connected with the robotic arm 20 and the surgical tool 30, respectively. At least one second deformable membrane 200 is sealingly provided on the adapter substrate 100 to cover at least one transmission window. It is to be understood that the circumference of the second deformable membrane 200 may be sealingly connected (e.g. welded, bonded, etc.) to the adapter substrate 100. By covering at least one transmission window, an effective barrier, for example, a sterile barrier against bacteria, is formed between the first side and the second side of the adapter substrate 100.

The at least one transmission member 300 is sealingly provided on the second deformable membrane 200 and is located in the at least one transmission window. For example, the transmission member 300 may be integrally molded, welded, or bonded with the second deformable membrane 200 so that there is no gap between the connecting locations of the transmission member 300 and the second deformable membrane 200 to sealingly isolate the contaminated area and the sterile area. The transmission member 300 may include a first interface 310 located at a first side of the second deformable membrane 200 and a second interface 320 located at a second side of the second deformable membrane 200. It is to be understood that the first side and the second side may be the front side and the rear side of the adapter substrate 100 when expanded, or the inner side and the outer side of the adapter substrate 100 in the folded state.

The at least one transmission member 300 is configured for transmission between the first interface 310 and the second interface 320 through deformation of the at least one second deformable membrane 200. For example, the first interface 310 is configured to couple with the connecting interface 331 of the surgical tool 30, and the second interface 320 is configured to couple with the driving interface 211 of the driving device. The at least one transmission member 300 is configured to drive the at least one connecting interface 331 to linearly move through deformation of the at least one second deformable membrane 200 under linear driving of the driving interface 211. The at least one connecting interface 331 drives the at least one sliding block 33 to linearly move through deformation of the at least one first deformable membrane 37.

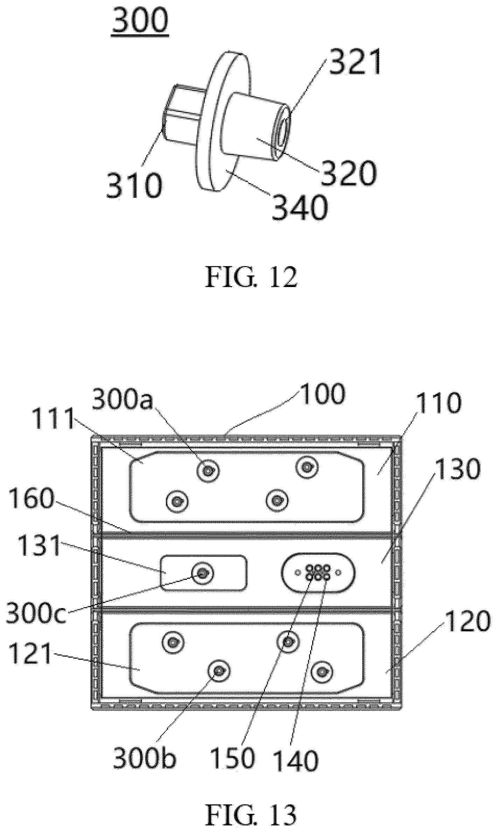

FIGs. 11 and 12 show structural schematic diagrams of a first side and a second side of the transmission member 300 according to some embodiments of the present disclosure, respectively. As shown in FIGs. 11 and 12, the transmission member 300 may include a first interface 310 and a second interface 320 located at both ends and fixedly connected. The first interface 310 and the second interface 320 are respectively provided with coupling structures, for example, coupling grooves 311 and 321, side section 312, and the like. It is to be understood that the structures of the first interface 310 and the second interface 320 may be the same or different, for example, the first interface 310 may have an irregular cylindrical shape (for example, including the side section 312), and the second interface 320 may have a cylindrical shape or a round-stage shape. Assembly may be facilitated by setting interfaces to have different structures.

In some embodiments, the coupling structure of the first interface 310 of the transmission member 300 may include a protrusion. The connecting interface 331 may include a corresponding groove. The second interface 320 of the transmission 300 may include a groove. The driving interface 211 may include a corresponding protrusion. The protrusion engages with the groove to realize coupling of the driving interface 211 of the robotic arm 20 and the connecting interface 311 of the surgical tool 30 through the transmission member 300. The above is merely an example. One of the interface of the transmission member 300 (e.g., the first interface 310 or the second interface 320) and the driving interface 211 may be a protrusion and the other may be a groove. Alternatively, the transmission member 300 and the driving interface 211 may be other structures enabling connection with each other.

In some embodiments, as shown in FIGs. 3 and 8, the connecting interface 331 may include a groove. The groove is recessed radially inward along the housing and extends in a direction perpendicular to the length of the housing. The groove is provided with an opening toward the third side (e.g., the lower side plate 352) of the housing. For example, the cross-section of the groove may be similar to a "U" shaped structure. In some embodiments, the opening of the groove is in a closing up state from the opening end in a direction away from the lower side plate 352. For example, the opening size may be gradually reduced, in a bell-mouth or funnel-like shape. In this way, during mounting of the surgical tool 30 to an external device, for example, a connecting adapter, it is easier for the first interface 310 to enter the groove interior of the connecting interface 331 through the opening.

In some embodiments, the transmission member 300 may further include an extension 340 circumferentially protruding outward. The second deformable membrane 200 may be sealingly connected with the extension 340. Providing the extension may make the sealing connection between the second deformable membrane and the transmission member more stable and reliable.

FIG. 13 shows a simplified front view of a connecting adapter 10 in an expanded state according to some embodiments of the present disclosure. For convenience of illustration, the shadow indicating the second deformable membrane 200 is omitted in FIG. 13, and only the transmission member 300 is retained. As shown in FIG. 13, the at least one transmission window may include a first transmission window 111 and a second transmission window 121. In some embodiments, as shown in FIG. 10, the adapter substrate 100 may be in a Chinese character " 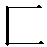 " shape or may be folded to form a " 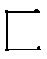 " shape. The adapter substrate 100 may include a left side substrate 110, a right side substrate 120, and a middle substrate 130. For example, the first transmission window 111 and the second transmission window 121 may be provided on the left side substrate 110 and the right side substrate 120, respectively. In some embodiments, the at least one transmission window may include a third transmission window 131, which may be provided on the middle substrate 130. It may be understood by those skilled in the art that the left side, the middle and the right side referred to herein are for the convenience of representing the relative positional relationship, and should be interpreted broadly, and other namings, for example, the upper side, the middle and the lower side, and the like may also be adopted.

In some embodiments, as shown in FIG. 13, the at least one transmission member 300 may include at least one first transmission member (e.g. a set of transmission members 300a) located within the first transmission window 111, and at least one second transmission member (e.g. a set of transmission members 300b) located within the second transmission window 121. In some embodiments, the at least one transmission member 300 may include at least one third transmission member (e.g. a set of transmission members 300c) located within the third transmission window 131. In some embodiments, the first transmission window 111 and the at least one transmission member 300a are arranged mirror-symmetrically with respect to the second transmission window 121 and the at least one transmission member 300b. It is to be understood that a set of transmission members 300a and a set of transmission members 300b may respectively include a plurality of transmission members spaced apart, and a set of transmission members 300c may include one transmission member. As shown in FIG. 13, there may be four transmission members located within the first transmission window 111 and the second transmission window 121, respectively. The four transmission members 300a located within the first transmission window 111 are arranged mirror-symmetrically with respect to the four transmission members 300b located within the second transmission window 121 in the direction of linear movement (for example, the direction of the length of the adapter substrate 100). In this way, the cooperative driving of the transmission members located within the first transmission window 111 and the second transmission window 121 may be facilitated. The above numbers are merely an example. The number of transmission members may also be six, eight, or the like. The number of transmission members may be adjusted according to the number of connecting interfaces on the external device to be driven, for example, a surgical tool.

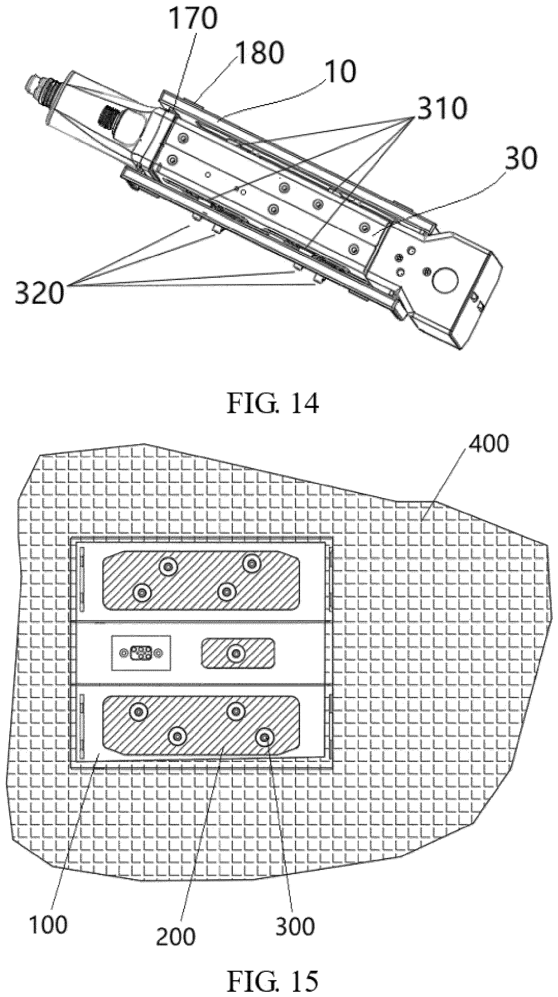

FIG. 14 shows a structural schematic diagram of a connecting adapter 10 assembling with a surgical tool 30 according to some embodiments of the present disclosure. As shown in FIG. 14, the proximal end of the surgical tool 30 may be mounted in the connecting adapter 10 in a direction perpendicular to the middle substrate 130 of the connecting adapter 10 (for example, the direction into the page as shown in FIG. 14). The lower side plate 352 of the surgical tool 30 abuts on the middle substrate 130 of the adapter substrate 100. The communication contacts 140 on the middle substrate 130 are communicatively connected with the communication interface 357. A housing window (e.g., housing windows 3531, 3521) on the housing 35 of the surgical tool 30 extends along the direction of the length of the housing 35. The connecting interface (e.g., connecting interface 331 shown in FIGs 3 and 8) is exposed from the housing window and may translate along the housing window. The inner sides of the left side substrate 110 and the right side substrate 120 of the connecting adapter 10 are provided with a first interface 310. The first interface 310 is coupled with the connecting interface 331 through a coupling structure on the connecting interface 331 to transmissibly connect the surgical tool 30 with the connecting adapter 10. The outer side of the proximal end of the surgical tool 30 is provided with a stop structure (e.g. the stop structure 38 shown in FIG. 3) along a direction perpendicular to the middle substrate 130 of the connecting adapter 10. The inner sides of the left side substrate 110 and/or the right side substrate 120 are provided with at least one stop portion 170 (shown in FIG. 10) protruding from the surface of the left side substrate 110 and/or the right side substrate 120. The stop structure 38 engages with the stop portion 170 to restrict the movement of the surgical tool 30 along the direction of the length to detachably connect the surgical tool 30 with the connecting adapter 10.

In some embodiments, as shown in FIGs. 9 and 13, the adapter substrate 100 is further provided with at least one communication contact 140 for forming a communication connection between the first side and the second side of the adapter substrate 100. For example, the first side and the second side of the adapter substrate 100 are connected with the surgical tool 30 and the robotic arm 20, respectively. The at least one communication contact 140 may be used to communicatively connect with the surgical tool 30 and the robotic arm 20 to form a communication connection between the surgical tool 30 on the first side and the robotic arm 20 on the second side of the adapter substrate 100. As shown in FIGs. 9 and 13, a plurality of communication contacts 140 arranged in a matrix may be provided on the middle substrate 130. The surgical tool 30 and the robotic arm 20 are provided with a plurality of conduction points thereon correspondingly, for example, the communication interface 357 provided on the lower side plate 352 of the surgical tool 30. After the mounting of the surgical tool 30 and the robotic arm 20 is completed, the plurality of communication contacts 140 are conducted with the conduction points to form a communication connection between the surgical tool 30 and the robotic arm 20. It is to be understood by those skilled in the art that an assembler may determine whether the adapter substrate 100, the surgical tool 30 and the robotic arm 20 are mounted in place by determining whether the communication contacts form a communication connection with the conduction points. It is also possible to read parameter information on the surgical tool 30 and the robotic arm 20 or to write parameter information to the surgical tool 30 and the robotic arm 20 through a communication connection formed by the communication contacts and the conduction points.

As shown in FIG. 13, in some embodiments, at least one ground pin 150 is further provided on the adapter substrate 100. Two ground pins 150 may be provided on the middle substrate 130. Electrostatic damage to the components of the system are prevented by providing ground pins.

In some embodiments, as shown in FIG. 10, the inner sides of the left side substrate 110 and/or the right side substrate 120 are provided with at least one stop portion 170 protruding from the surface of the left side substrate 110 and/or the right side substrate 120. It is to be understood that the inner sides of the left side substrate 110 and the right side substrate 120 refer to the sides facing each other when the left side substrate 110 and the right side substrate 120 are arranged opposite each other. The outer sides of the left side substrate 110 and the right side substrate 120 refer to the sides facing away from each other when the left side substrate 110 and the right side substrate 120 are arranged opposite each other. As shown in FIG. 10, two stop portions 170 may be provided on the inner sides of the left side substrate 110 and the right side substrate 120, respectively. For example, the stop portion 170 may be a protrusion. The outer side of the housing of the surgical tool 30 is provided with a stop groove (e.g. the stop structure 38) mating with the stop portion 170. The stop portion 170 engages with the stop structure 38 to restrict movement of the surgical tool in a direction of linear movement, for example, the direction of the length of the middle substrate 130.

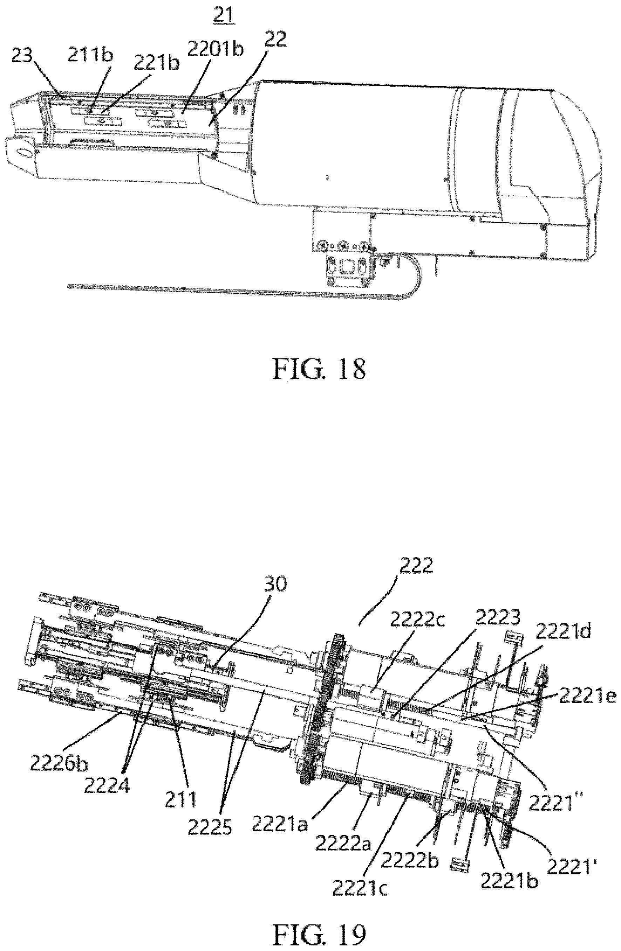

As shown in FIG. 10, in some embodiments, the outer sides of the left side substrate 110 and/or the right side substrate 120 are correspondingly provided with at least one connecting structure, e.g. a connection protrusion 180 for engaging with a groove (e.g. the groove 23 shown in FIG. 18) on the robotic arm 20. It is to be understood that the outer sides of the left side substrate 110 and the right side substrate 120 may be provided with two connecting protrusions 180, respectively. The adapter substrate 100 and the robotic arm 20 are detachably connected by engaging the connecting protrusion 180 with the groove 23. It is to be understood by those skilled in the art that the stop portion 170 and the connecting protrusion 180 shown in FIG. 10 are only exemplary embodiments, and other structures for example, grooves, magnetic attraction structures, and the like, may be adopted. The stop portion 170 or the connecting structure 180 may also be a groove. A corresponding protrusion is provided on the surgical tool 30 or the robotic arm 20 to enable the detachable fixed connection between the adapter substrate 100 and the surgical tool 30 or the robotic arm 20. It is to be understood that the connecting structure may also be other structures enabling a detachable connection.

In some embodiments, as shown in FIG. 10, the outer sides of the left side substrate 110 and/or the right side substrate 120 are further provided with a shielding portion 190 extending outwardly from the substrate surfaces. The shielding portion 190 may be configured to shield a portion of the transmission member 300 exposed on the outer sides of the substrates. For example, the shielding portion 190 may be provided at an upper end (the upper end as shown in FIG. 10) and/or a side end (the left and right ends as shown in FIG. 10) of the left side substrate 110 and the right side substrate 120. The length of the shielding portion 190 extending outward is greater than or equal to the length of the transmission member 300 exposed on the outer side of the transmission window. The shielding portion 190 may extend outward perpendicularly to the substrate surface or may extend outward at an included angle with respect to the substrate surface. By providing the shielding portion, a motion space for linear movement of the transmission member 300 may be formed between the outer side of the adapter substrate 100 and the robotic arm 20 after the mounting of the adapter substrate 100 and the robotic arm 20 is completed.

FIG. 15 shows a rear view of a connecting adapter 10 in an expanded state according to some embodiments of the present disclosure. As shown in FIG. 15, in some embodiments, the at least one connecting adapter 10 further includes a sterile protective membrane 400. The sterile protective membrane 400 is sealingly connected with the circumferential edge of the adapter substrate 100 and extends outwardly. It is to be understood that the adapter substrate 100 may have a " 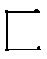 " shape or a foldable sheet structure, and the sterile protective membrane 400 is circumferentially connected (e.g. welded or bonded) with the adapter substrate 100 and extends outwardly to cover at least a portion of the robotic arm 20. It is to be understood that the sterile protective membrane 400 may be a TPU membrane, so as to facilitate sterilization during manufacturing to achieve medical material grade. The adapter substrate 100 may be plastic, so as to facilitate connection with the sterile protective membrane 400 and the second deformable membrane 200. It is to be understood that the extended sterile protective membrane 400 may be shaped to fit the portion of the robotic arm 20 that needs to be covered. By covering the robotic arm 20 with the sterile protective membrane 400, the surgical tool 30 and the robotic arm 20 may be isolated to isolate the contaminated side and the sterile side, meeting the requirements of the operating environment.

In some embodiments, as shown in FIG. 13, the adapter substrate 100 has a foldable sheet structure. The adapter substrate 100 may also be provided with at least one folding line 160 thereon, with the at least one folding line 160 extending along the direction of the length of the middle substrate 130. The adapter substrate 100 may be folded along the folding line 160 to form a " 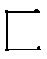 "shape. As shown in FIG. 13, the adapter substrate 100 may be provided with two folding lines 160 thereon to divide the adapter substrate 100 into a left side substrate 110, a right side substrate 120, and a middle substrate 130. The folding line 160 makes it easy for an operator to fold the adapter substrate 100 along the folding line to form a " 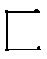 " shape with simple and convenient operation.

In some embodiments, the second deformable membrane 200 includes a sheet membrane adapted to the shape of the adapter substrate 100. The sheet membrane is sealingly provided on the surface of the adapter substrate 100 to cover the at least one transmission window. For example, as shown in FIG. 9, the adapter substrate 100 may be rectangular, and the second deformable membrane 200 may be a rectangular sheet membrane adapted to the adapter substrate 100. The second deformable membrane 200 is connected with the circumferential edge of the adapter substrate 100, so that the second deformable membrane 200 is affixed to the surface of the adapter substrate 100 to cover a plurality of transmission windows on the adapter substrate 100. In some embodiments, the second deformable membrane 200 may also be connected with the adapter substrate 100 at the folding lines 160, so as to maintain the second deformable membrane 200 and the adapter substrate 100 in close contact and achieve a good seal for the transmission window with the adapter substrate 100 folded.

In some embodiments, the second deformable membrane 200 includes at least one sheet membrane corresponding to at least one transmission window (e.g., transmission windows 111, 121, 131), respectively, and the at least one sheet membrane is respectively sealingly connected with the adapter substrate 100 to cover the at least one transmission window. It is to be understood that the at least one transmission window (e.g., transmission windows 111, 121, 131) may be rectangular, polygonal, circular, other regular or irregular shapes, or a combination of multiple shapes, or the like. The second deformable film 200 may include sheet membranes, the number of which is consistent with the number of transmission windows. The shapes of the sheet membranes may match the shapes of individual transmission windows. Each sheet membrane is connected with the circumferential edge of each transmission window, respectively, to cover each transmission window.

The deformable membranes (e.g., the first deformable membrane 37, the second deformable membrane 200) may comprise various deformable materials, for example, elastic membranes. As an example, the deformable membrane may include, for example, a rubber membrane (e.g., a TPU membrane), a plastic membrane, and the like. The deformable membrane may expand and contract with the linear motion of the transmission member to ensure that the transmission member will not tear and damage the deformable membrane during the motion.

The first deformable membrane 37 covers at least one housing window of the surgical tool 30 to form a sterile barrier between the outer side (a sterile side) and the inner side (possibly a contaminated side) of the surgical tool 30 that effectively blocks bacteria. The second deformable membrane 200 covers at least one transmission window to form a sterile barrier between the inner and outer sides of the connecting adapter 10 that effectively blocks bacteria. The blocking effect may be further ensured through double barriers. A sterile surgical operation environment is provided, avoiding bacteria contamination of the surgical tool, by covering the driving device 21 and the robotic arm 20 with the sterile protective membrane 400, so that a sterile barrier that effectively blocks bacteria is formed between the portion of the robotic arm close to the surgical tool 30 and the surgical tool 30. At least one transmission member 300 is provided on the second deformable membrane 200, and the driving (e.g., linear driving) of the first side of the connecting adapter 10 is transferred to the second side of the connecting adapter 10 through the transmission member 300 through deformation of the second deformable membrane 200 to achieve direct transfer of various motions, e.g., linear motions. Through the deformation of the first deformable membrane 37, the driving interface of the external device is allowed to drive the connecting interface to move to enable the push and/or pull of the driving wire, thereby driving the surgical tool to perform various operations, so that the structure is simple and easy to operate.

The present disclosure also provides a connecting assembly including a first external device, a second external device, and a connecting adapter in any of the embodiments of the present disclosure, e.g., the connecting adapter 10 shown in FIGs. 1-5. The first external device may include at least one connecting interface, the second external device may include at least one driving interface, and the first interface 310 and the second interface 320 of the transmission member 300 of the connecting adapter 10 are coupled with the connecting interface and the driving interface, respectively. In some embodiments, the first external device may include the surgical tool 30 and the second external device may include the driving device 21. It is to be understood that the first external device and the second external device may also be other devices with interfaces. For example, the first interface 310 of the transmission member 300 may include a protrusion. The connecting interface may include a corresponding groove. The second interface 320 of the transmission member 300 may include a groove. The driving interface may include a corresponding protrusion. The protrusion is engaged with the groove to realize coupling of the connecting interface of the first external device and the driving interface of the second external device through the transmission member 300 to transfer the driving of the second external device to the first external device. The above is only an example. One of the interface of the transmission member 300 and the interface of the external device may be a protrusion and the other may be a groove. Alternatively, the interfaces of the transmission member and the external device may also be other structures enabling connection with each other.

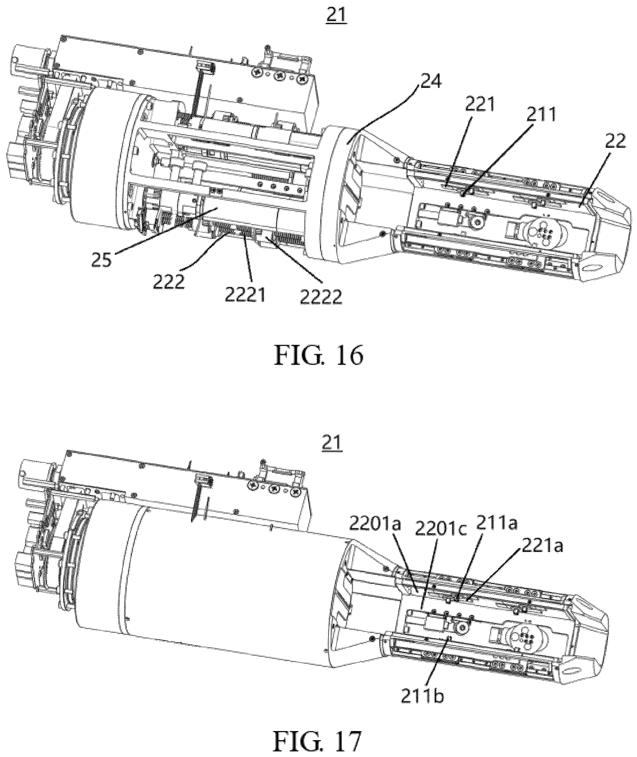

In some embodiments, the second external device may further include at least one motor and at least one transmission mechanism (e.g., transmission mechanism 222 shown in FIG. 16). The motor is connected with the transmission mechanism. The driving interface of the second external device is fixedly provided on the transmission mechanism. The transmission mechanism is configured to convert the rotational motion of the motor into linear motion to drive the linear motion of the at least one transmission member 300, so as to drive the linear motion of the connecting interface of the first external device through the deformation of the at least one second deformable membrane.

The present disclosure provides a driving device. FIG. 16 shows a structural schematic diagram of the interior of a driving device 21 according to some embodiments of the present disclosure. The driving device 21 may include at least one motor 25, at least one transmission mechanism 222 and at least one driving interface 211. The at least one transmission mechanism 222 is coupled with the at least one motor 25. The transmission mechanism 222 is configured to convert the rotational motion of the motor 25 into linear motion. The at least one driving interface 211 is connected with the at least one transmission mechanism 222. The driving interface 211 is configured for coupling with an external device (e.g. the connecting adapter 10), and to linearly move under the driving of the transmission mechanism 222, providing driving to the connecting adapter 10. The driving device 21 may directly provide linear driving to the connecting adapter 10 through the driving interface 211 to drive a linear motion of the transmission member 300, and the structure is simple.

In some embodiments, as shown in FIG. 16, the driving device 21 may further include a body 24. The body 24 includes a receiving slot 22 provided at a distal end, and at least one driving window 221 provided on an inner wall of the receiving slot 22, wherein at least one driving interface 211 is located within the at least one driving window 221. It is to be understood that the body 24 may include a housing of the driving device 21 or a frame of the driving device, and the shape of the body 24 may be set according to the layout of the driving device 21 to cover or carry the internal structure of the driving device 21. For example, as shown in FIG. 16, the body 24 may include a proximal end portion and a distal end portion, wherein the distal end portion is smaller in size than the proximal end portion, and may be connected with the proximal end portion via a round-stage-like structure. The motor 25 and the transmission mechanism 222 may be provided within the proximal end portion of the body 24, and the receiving slot 22 may be provided on the distal end portion of the body 24.

The shape of the receiving groove 22 may include, but is not limited to, a cube, a cylinder, a round stage, an irregular three-dimensional structure, or the like, or a combination thereof. The driving interface 211 may be located in the driving window 221 and protrude from the driving window 221, and move within the driving window 221 under the driving of the transmission mechanism 222. It is to be understood that coupling structures, for example, coupling grooves, coupling protrusions, side sections, and the like, are provided on the driving interface 211. The structures of the driving interface 211 may be the same or different. For example, the interface may have an irregular cylindrical shape (for example, including a side section), or the interface may have a cylindrical shape or a round stage shape, or the like. Assembly may be facilitated by setting interfaces to have different structures.

The connecting adapter 10 is detachably connected with the receiving slot 22 of the driving device 21. The surgical tool 30 is detachably connected with the connecting adapter 10. The surgical tool 30 is detachably connected within the receiving slot 22 of the driving device 21 via the connecting adapter 10.

FIGs. 17 and 18 show structural schematic diagrams, from different perspectives, of a driving device according to some embodiments of the present disclosure, respectively. For clarity of illustration, the distal end of the housing in FIG. 17 has a portion of the enclosure omitted, showing the internal structure of the distal end, and the enclosure at the distal end is not omitted in FIG. 18. In some embodiments, as shown in FIGs. 17 and 18, the at least one driving window 221 may include at least one first driving window 221a located on the first inner wall of the receiving slot 22 and at least one second driving window 221b located on the second inner wall of the receiving slot 22. The at least one driving interface 211 includes at least one first driving interface 211a located within the first driving window 221a and at least one second driving interface 211b located within the second driving window 221b. In some embodiments, the first inner wall and the second inner wall are opposite to each other, as shown in FIGs. 17 and 18. For example, the receiving slot 22 may have a cubic structure. The receiving slot 22 may include a left side inner wall 2201a, a right side inner wall 2201b, and a middle inner wall 2201c. The first driving window 221a and the second driving window 221b may be provided on the left side inner wall 2201a and the right side inner wall 2201b, respectively.

In some embodiments, the at least one driving window 211 may further include at least one third driving window (not shown) located on the third inner wall (e.g., the middle inner wall 2201c) of the receiving slot 22, and the at least one driving interface 211 may further include at least one third driving interface (not shown) located within the third driving window. It is to be understood by those skilled in the art that the left side, the middle and the right side referred to herein are for the convenience of representing the relative positional relationship, and should be interpreted broadly, and other namings, for example, the front side, the bottom and the rear side, and the like may also be adopted.

In some embodiments, as shown in FIGs. 17 and 18, the driving device 21 further includes at least one engaging structure, e.g., the engaging groove 23, provided on the first inner wall (e.g., the left side inner wall 2201a) and/or the second inner wall (e.g., the right side inner wall 2201b), and the engaging groove 23 is configured for engaging with an engaging protrusion (e.g., the engaging protrusion 180 shown in FIG. 14) on an external device (e.g., the connecting adapter 10 shown in FIG. 14). It is to be understood that the left side inner wall 2201a and the right side inner wall 2201b may be provided with two engaging grooves 23, respectively. The engaging groove 23 is engaged with the engaging protrusion 180 to detachably connect the driving device 21 with the connecting adapter 10.

It is to be understood by those skilled in the art that the engaging groove 23 shown in FIG. 18 is only an exemplary embodiment, and other structures, for example, protrusions, magnetic attraction structures, and the like, may be adopted. The engaging structure may also be a protrusion, and a corresponding groove is provided on the external device to enable the detachable connection of the driving device 21 with the external device (e.g. the connecting adapter 10). It is to be understood that the engaging structure may also be other structures enabling a detachable connection.

FIG. 19 shows a structural schematic diagram of a transmission mechanism 222 mating with an external device (e.g. the surgical tool 30) according to some embodiments of the present disclosure. As shown in FIGs. 16 and 19, the at least one transmission mechanism 222 may include a screw 2221 (e.g., screw 2221', 2221"), at least one nut 2222 (e.g., nuts 2222a-2222c), and a plurality of balls (not shown). A plurality of balls are provided between the screw 2221 and the at least one nut 2222. The friction between the nut 2222 and the screw 2221 may be changed to rolling friction through the ball, so as to reduce the friction loss of the transmission mechanism 222 when it is in motion, and the service life of the transmission mechanism 222 may be improved. The nut 2222 may be connected (e.g., directly or indirectly) with the driving interface 211. The screw 2221 is coupled with the at least one motor 25 and rotates under the driving of the motor 25 to drive the nut 2222 to linearly move along the screw 2221 to drive the driving interface 211 to linearly move. It is to be understood that the transmission mechanism 222 may also not include balls. For example, as shown in FIG. 16, the transmission mechanism 222 includes a screw 2221 and at least one nut 2222 threadedly connected with the screw 2221. The nut 2222 may be connected with the driving interface 211 through a connecting rod (e.g. the connecting rod 2225). The screw 2221 is coupled with the at least one motor 25 and rotates under the driving of the motor 25 to drive the nut 2222 to linearly move along the screw to drive the driving interface 211 to linearly move. It is to be understood that the number of transmission mechanisms 222 may be adjusted according to the number of connecting interfaces of external devices.

In some embodiments, as shown in FIG. 19, the at least one nut 2222 may include a nut 2222a and a nut 2222b. The screw 2221 may include at least one section of thread. For example, the screw 2221 includes a screw 2221' with two sections of thread. In some embodiments, screw 2221' includes thread 2221a and thread 2221b in the opposite direction of thread 2221a, and nuts 2222a and 2222b are provided on thread 2221a and thread 2221b, respectively. For example, the threads 2221a and 2221b may be oblique threads having opposite thread directions, and the nuts 2222a and 2222b are provided with oblique threads mating with the thread 2221a and thread 2221b. When the motor drives the screw 2221' to rotate, the nut 2222a and the nut 2222b move linearly in opposite directions at the same speed due to the opposite helical directions of the thread 2221a and thread 2221b, to achieve cooperative reverse driving of a pair of driving interfaces 211. It is to be understood that the length of the thread 2221a and thread 2221b, the sizes of the thread gap of the thread 2221a and thread 2221b, and the like, may be set according to the range requirement of the actual driving interface.

In some embodiments, as shown in FIG. 19, the screw 2221' may also include a non-threaded segment 2221c between the thread 2221a and the thread 2221b. For example, the non-threaded segment 2221c may be located at the middle portion of the screw 2221'. The non-threaded segment 2221c may prevent the nut 2222a and the nut 2222b from moving beyond the corresponding threaded regions when they are in linear motion along the screw 2221' when the screw 2221' is rotated. It is to be understood that the screw 2221' may also include non-threaded segments located at both ends of the thread 2221a and the thread 2221b.

In some embodiments, the screw 2221 may also include a single threaded screw 2221". As shown in FIG. 19, the screw 2221" may include a threaded segment 2221d at the distal end and a non-threaded segment 2221e at the proximal end. The at least one nut 2222 may include a nut 2222c provided on the threaded segment 2221d of the screw 2221".

In some embodiments, the nut 2222a and the nut 2222b of the two-threaded-segment screw 2221' may be located on two sides corresponding to the two side inner walls 2201a and 2201b of the receiving slot 22 to drive the driving sliding blocks 2224 located at the two side inner walls 2201a and 2201b of the receiving slot 22. The nut 2222c may be located at the bottom side corresponding to the middle inner wall 2201c of the receiving slot 22 to drive the driving sliding block 2224 located at the bottom side inner wall 2201c of the receiving slot 22. The arm body 31 may be driven to bend through the nut 2222a and the nut 2222b. Opening and closing of the end instrument 32 may be driven through the nut 2222c.

In some embodiments, as shown in FIG. 19, the transmission mechanism 222 may further include at least one connecting sliding block 2223 (one of which is shown), at least one driving sliding block 2224, and at least one connecting rod 2225. The at least one connecting sliding block 2223 is fixedly connected with the at least one nut 2222 (e.g. nuts 2222a-2222c). The at least one driving interface 211 may be connected with the at least one driving sliding block 2224. The proximal end of the at least one connecting rod 2225 is fixedly connected with the at least one connecting sliding block 2223, and the distal end of the at least one connecting rod 2225 is fixedly connected with the at least one driving sliding block 2224. As shown in FIGs. 8 and 19, the driving interface 211 is fixedly provided on the driving sliding block 2224. The connecting rod 2225 drives the driving sliding block 2224 to linearly move, to drive the driving interface 211 to linearly move through the driving sliding block 2224. The connecting rod 2225 may be a single connecting rod or a combination of a plurality of connecting rods. The connecting rod 2225 may be a straight rod, or may be a rod of other shape, such as an L-shape, a curved shape, or the like. By setting the shape of the connecting rod 2225, the number of connecting rods, and the combination form of connecting rods, it is possible to accommodate different structural arrangements of the driving device 21 to adjust the driving interface 211 to a position that facilitates coupling with external devices (e.g. the adapter 10 and the surgical tool 30). In this way, the structure of the driving device 21 may be reduced in size and the use of internal space of the driving device 21 may be maximized.

It is to be understood that at least one first sliding rail (not shown) and at least one second sliding rail 2226b may also be provided within the driving device 21. The connecting sliding block 2223 and the driving sliding block 2224 may be provided on the first sliding rail and the second sliding rail 2226b, respectively. By providing sliding rails, the motion of the sliding block is more stable and reliable, so that the motion of the driving interface 211 is more stable. Thus, the accuracy of driving transmission is improved. It is to be understood that the sliding rails in the present disclosure should be interpreted broadly and may include various structures, for example, sliding grooves, sliding rods, and the like.

In some embodiments, the nut 2222a and the nut 2222b located on the same screw 2221 (e.g., screw 2221') may be connected with an pair of adjacent connecting sliding blocks 2223, respectively, for example, connected with a pair of connecting sliding blocks 2223 located on the same first sliding rail. The pair of connecting sliding blocks 2223 may be connected with an pair of adjacent connecting rods 2225. The pair of connecting rods 2225 may be connected with an pair of adjacent driving sliding blocks 2224, for example, connected with a pair of driving sliding blocks 2224 located on the same second sliding rail 2226b.

It is to be understood that the at least one driving device 21 is connected with the driving interface 211 via a connecting rod 2225 (e.g., via the driving sliding block 2224 and the sliding rail 2226b). The driving sliding block 2224 may move linearly, e.g., move reciprocally, along the corresponding driving windows (e.g., the first driving window 221a and the second driving window 221b) to drive the driving interface 211 connected with the driving sliding block 2224 to move reciprocally along the driving windows. The driving interface 211 is coupled with the transmission member 300 of the connecting adapter 10, to drive the transmission member 300 to move reciprocally along transmission windows (e.g., the transmission windows 111, 121, 131), to drive the sliding block 33 to move reciprocally through the transmission member 300, to push and/or pull the driving wire 34. In this way, the linear driving of the driving device 21 is directly transferred to the surgical tool 30 via the connecting adapter 10, to control the motion of the surgical tool 30.

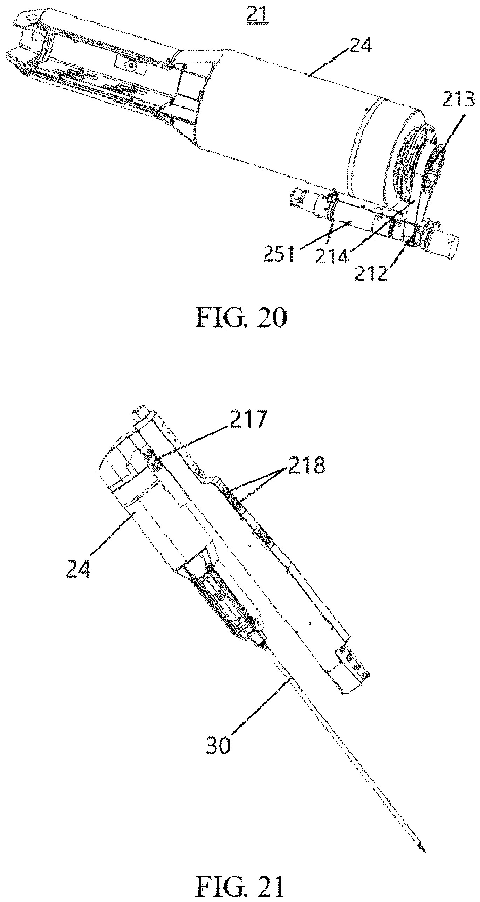

In some embodiments, the driving device 21 may further include an integral rotation mechanism. FIG. 20 shows a structural schematic diagram of an integral rotation mechanism according to some embodiments of the present disclosure. In some embodiments, as shown in FIG. 20, the integral rotation mechanism may include a driving wheel 212, a driven wheel 213, and a synchronous belt 214 linked with the driving wheel 212 and the driven wheel 213. Among them, at least one motor 25 includes a rotation mechanism motor 251, the driving wheel 212 is fixedly connected with the output end of the rotation mechanism motor 251, the driven wheel 213 is fixedly connected with the body 24, the synchronous belt 214 moves along with the driving wheel 212 and the driven wheel 213. The driving wheel 212 is rotated under the driving of the rotation mechanism motor 251, the synchronous belt 214 drives the driven wheel 213 to move together, and the driven wheel 213 drives the body 24 to rotate, so as to realize the overall rotation of the driving device 21.

In some embodiments, the integral rotation mechanism may employ other structure, for example, including a first gear and a second gear. The outer circumferential surface of the first gear is fixedly connected with the body, and the second gear is meshed with the inner circumferential surface of the first gear, wherein the at least one motor includes a rotation mechanism motor, and the second gear is coupled with the output end of the rotation mechanism motor for driving the first gear to rotate under the driving of the rotation mechanism motor. The overall rotation of the driving device may be realized by rotation of the first gear driving the body to rotate. The overall rotation of the external device is driven by the overall rotation of the driving device.

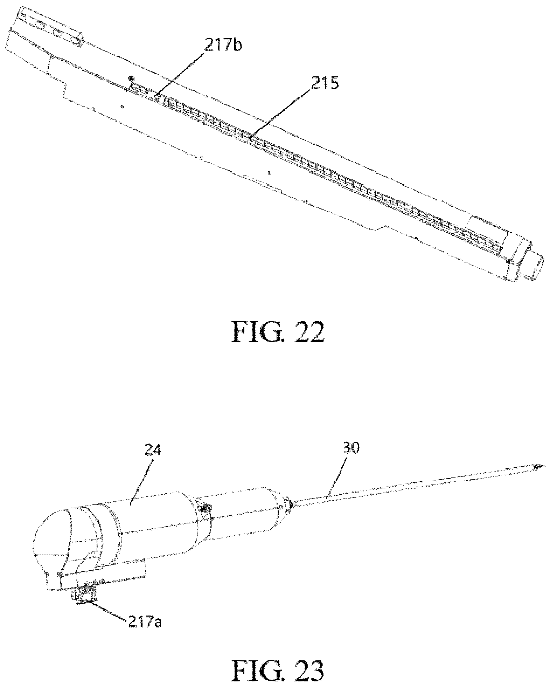

FIGs. 21, 22, and 23 shows a structural diagram of a linear mechanism mating with a driving device, a structural diagram of a linear mechanism, and a partial structural diagram of a driving device according to some embodiments of the present disclosure, respectively. In some embodiments, as shown in FIGs. 21, 22 and 23, the driving device 21 may further include a linear mechanism. The linear mechanism may include a screw 215, a mechanism sliding block 217, and a sliding rail (not shown). The mechanism sliding block 217 is slidably connected with the screw 215 and slidably provided on the sliding rail. The body 24 is fixedly provided on the mechanism sliding block 217. It is to be understood that the mechanism sliding block 217 may include a mechanism sliding block 217a fixedly connected with the body 24, and a mechanism sliding block 217b slidably connected with the screw 215 and slidably provided on the sliding rail, and the mechanism sliding block 217a is fixedly connected with the mechanism sliding block 217b. Among them, at least one motor 25 includes a linear mechanism motor (not shown in the figure), the screw 215 is coupled with the output end of the linear mechanism motor, and drives the mechanism sliding block 217 to slide along the sliding rail under the driving of the linear mechanism motor. The body 24 is driven to linearly move through the mechanism sliding block 217.

In some embodiments, as shown in FIG. 21, at least one function button 218 may be provided on the linear mechanism for the operator to manually press the button 218 to control the linear feed and retreat motion of the linear mechanism. For example, the button 218 may include a forward button and a reverse button arranged separately. By a linear mechanism controlling feeding and retreating of an external device (e.g. the surgical tool 30) on the driving device 21, the surgical tool 30 is controlled to reach the surgical operation area.

The following embodiments are also disclosed by the present disclosure:
Embodiment 1. A connecting adapter, comprising:
   an adapter substrate comprising at least one transmission window;
   at least one second deformable membrane sealingly provided on the adapter substrate to cover the at least one transmission window;
   at least one transmission member sealingly provided on the second deformable membrane and located in the at least one transmission window, the transmission member comprising a first interface located on a first side of the second deformable membrane and a second interface located on a second side of the second deformable membrane, the at least one transmission member configured for transmission between the first interface and the second interface through deformation of the at least one second deformable membrane.
Embodiment 2. The connecting adapter according to embodiment 1, wherein the at least one transmission window comprises a first transmission window and a second transmission window, and the at least one transmission member comprises at least one first transmission member located within the first transmission window and at least one second transmission member located within the second transmission window.
Embodiment 3. The connecting adapter according to embodiment 2, wherein the first transmission window and the at least one first transmission member are arranged mirror-symmetrically with respect to the second transmission window and the at least one second transmission member.
Embodiment 4. The connecting adapter according to embodiment 2 or 3, wherein the adapter substrate comprises a left side substrate, a right side substrate and a middle substrate, and the first transmission window and the second transmission window are respectively provided on the left side substrate and the right side substrate.
Embodiment 5. The connecting adapter according to embodiment 4, wherein the at least one transmission window further comprises a third transmission window provided on the middle substrate.
Embodiment 6. The connecting adapter according to embodiment 4 or 5, further comprising at least one stop portion provided on an inner side of the left side substrate and/or the right side substrate and protruding from the left side substrate and/or the right side substrate.
Embodiment 7. The connecting adapter according to any of embodiments 4 to 6, further comprising at least one connecting structure provided on an outer side of the left side substrate and/or the right side substrate.
Embodiment 8. The connecting adapter according to any of embodiments 4 to 7, further comprising a shielding portion provided on an outer side of the left side substrate and/or the right side substrate and extending outwardly from the substrate surface, the shielding portion configured for shielding a portion of the transmission member exposed on the outer side of the adapter substrate.
Embodiment 9. The connecting adapter according to any of embodiments 1 to 8, further comprising:
   at least one communication contact provided on the adapter substrate, the at least one communication contact configured for forming a communication connection between the first side and the second side of the adapter substrate; and/or
   at least one ground pin provided on the adapter substrate.
Embodiment 10. The connecting adapter according to any of embodiments 1 to 9, wherein the adapter substrate is in a " 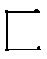 " shape; or
   the adapter substrate is provided with at least one folding line thereon, and the adapter substrate is foldable along the folding line to form a " 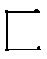 " shape.
Embodiment 11. The connecting adapter according to any of embodiments 1 to 10, further comprising: a sterile protective membrane sealingly connected with the circumferential edge of the adapter substrate and extending outwardly.
Embodiment 12. The connecting adapter according to any of embodiments 1 to 11, wherein the second deformable membrane comprises a sheet membrane adapted to the shape of the adapter substrate, and the sheet membrane is sealingly provided on the surface of the adapter substrate to cover the at least one transmission window; or
   the second deformable membrane comprises at least one sheet membrane respectively corresponding to the at least one transmission window, and the at least one sheet membrane is sealingly connected with the adapter substrate respectively to cover the at least one transmission window.
Embodiment 13. A connecting assembly, comprising:
   a first external device comprising at least one connecting interface;
   a second external device comprising at least one driving interface; and
   a connecting adapter according to any one of embodiments 1 to 12, wherein the first interface and the second interface of the transmission member of the connecting adapter are coupled with the connecting interface and the driving interface, respectively.
Embodiment 14: The connecting assembly according to embodiment 13, wherein the second external device further comprises at least one motor and at least one transmission mechanism, the motor is connected with the transmission mechanism, the driving interface is fixedly provided on the transmission mechanism, and the transmission mechanism is configured to convert rotational motion of the motor into linear motion to drive the at least one transmission member to linearly move, to drive the connecting interface to linearly move through deformation of the at least one deformable membrane.
Embodiment 15. A surgical robot system, comprising:
   at least one robotic arm; and
   a connecting assembly according to embodiment 13 or 14, wherein the second external device is arranged at a distal end of the robotic arm, the first external device comprises a surgical tool, and the robotic arm is connected with the surgical tool via the connecting adapter.
Embodiment 16. A driving device, comprising:
   at least one motor;
   at least one transmission mechanism coupled with the at least one motor, the transmission mechanism configured for converting rotational motion of the motor into linear motion; and
   at least one driving interface connected with the at least one transmission mechanism, the driving interface configured to be coupled with an external device and linearly move under the driving of the at least one transmission mechanism, providing driving to the external device.
Embodiment 17: The driving device according to embodiment 16, further comprising:
   a body comprising a receiving slot provided at a distal end and at least one driving window provided on an inner wall of the receiving slot, wherein the at least one driving interface is located within the at least one driving window.
Embodiment 18: The driving device according to embodiment 17, wherein the at least one driving window comprises at least one first driving window located on a first inner wall of the receiving slot and at least one second driving window located on a second inner wall of the receiving slot, and the at least one driving interface comprises at least one first driving interface located within the first driving window and at least one second driving interface located within the second driving window.
Embodiment 19: The driving device according to embodiment 18, wherein the at least one driving window further comprises at least one third driving window located on a third inner wall of the receiving slot, and the at least one driving interface further comprises at least one third driving interface located within the third driving window, wherein the first inner wall is opposite to the second inner wall.
Embodiment 20: The driving device according to embodiment 18 or 19, further comprising at least one engaging structure provided on the first inner wall and/or the second inner wall.
Embodiment 21: The driving device according to any one of embodiments 16 to 20, wherein the transmission mechanism comprises a screw, at least one nut, and a plurality of balls provided between the screw and the at least one nut, the nut connected with the driving interface, and the screw coupled with the motor and configured to rotate under the driving of the motor to drive the nut to linearly move along the screw to drive the driving interface to linearly move; or
   the transmission mechanism comprises a screw and at least one nut threadedly connected with the screw, the nut connected with the driving interface, the screw coupled with the motor and configured to rotate under the driving of the motor to drive the nut to linearly move along the screw to drive the driving interface to linearly move.
Embodiment 22: The driving device according to embodiment 21, further comprising:
   at least one connecting sliding block fixedly connected with the at least one nut;
   at least one driving sliding block, the at least one driving interface being connected with the at least one driving sliding block; and
   at least one connecting rod, a proximal end of which is fixedly connected with the at least one connecting sliding block and a distal end of which is fixedly connected with the at least one driving sliding block.
Embodiment 23: The driving device according to embodiment 22, wherein the at least one nut comprises a first nut and a second nut;
   the screw comprises a first thread and a second thread opposite to the direction of the first thread, and the first nut and the second nut are provided on the first thread and the second thread, respectively.
Embodiment 24: The driving device according to embodiment 23, wherein the first nut and the second nut positioned on the same screw are connected with an pair of adjacent connecting sliding blocks, an pair of adjacent connecting rods, and an pair of adjacent driving sliding blocks, respectively.
Embodiment 25: The driving device according to any one of embodiments 16 to 24, further comprising:
   an integral rotation mechanism comprising:
   a driving wheel, wherein the at least one motor comprises a rotating mechanism motor, the driving wheel being fixedly connected with an output end of the rotating mechanism motor;
   a driven wheel fixedly connected with the body; and
   a synchronous belt linked with the driving wheel and the driven wheel and configured for driving the body to rotate under the driving of the rotating mechanism motor; and/or
   a linear mechanism comprising a screw, a sliding rail and a mechanism sliding block, the mechanism sliding block being slidably connected with the screw and slidably provided on the sliding rail, and the body being fixedly provided on the mechanism sliding block, wherein the at least one motor comprises a linear mechanism motor, the screw is coupled with an output end of the linear mechanism motor and drives the body to slide along the sliding rail under the driving of the linear mechanism motor.
Embodiment 26. A surgical robot system, comprising:
   at least one robotic arm; and
   at least one driving device according to any one of embodiments 16 to 25, the at least one driving device being arranged at a distal end of the at least one robotic arm.
Embodiment 27: The surgical robot system according to embodiment 26, further comprising:
   at least one surgical tool comprising at least one connecting interface; and
   at least one connecting adapter configured for detachably connecting the surgical tool within a receiving slot of the driving device, the connecting adapter comprising at least one first interface for coupling with a connecting interface of the surgical tool and at least one second interface for coupling with the driving interface of the driving device.
Embodiment 28: The surgical robot system according to embodiment 27, the surgical tool comprising:
   an arm body;
   an end instrument arranged at a distal end of the arm body;
   a housing located at a proximal end of the arm body, the housing comprising at least one housing window;
   at least one first deformable membrane sealingly provided on the housing to cover the at least one housing window;
   at least one third sliding block slidably provided inside the housing, the connecting interface being connected with the at least one third sliding block, the connecting interface being sealingly connected with the first deformable membrane and located in the at least one housing window; and
   at least one driving wire for driving the arm body and/or the end instrument, a first end of the driving wire being fixedly connected with the third sliding block, and the connecting interface configured for receiving driving through deformation of the at least one first deformable membrane to drive the third sliding block to push or pull the driving wire.
Embodiment 29: The surgical robot system according to embodiment 27 or 28, the connecting adapter comprising:
   an adapter substrate comprising at least one transmission window;
   at least one second deformable membrane sealingly provided on the adapter substrate to cover the at least one transmission window;
   at least one transmission member sealingly provided on the second deformable membrane and located in the at least one transmission window, the transmission member comprising a first interface on a first side of the second deformable membrane for coupling with the connecting interface and a second interface on a second side of the deformable membrane for coupling with the driving interface, the transmission member receiving driving from the driving interface through deformation of the second deformable membrane.
Embodiment 30: The surgical robot system according to embodiment 29, wherein
   the inner wall of the receiving slot is provided with at least one engaging structure, the outer side of the adapter substrate is provided with at least one connecting structure adapted to the engaging structure, and the connecting structure is engaged with the engaging structure to detachably connect the adapter substrate with the robotic arm; and/or
   the inner side of the adapter substrate is provided with at least one stop portion protruding from the surface, the outer side of the proximal end of the surgical tool is provided with at least stop member, and the stop portion is engaged with the stop member to restrict the movement of the surgical tool in the axial direction.
Embodiment 31: The surgical robot system according to embodiment 29 or 30, wherein
   the at least one connecting adapter further comprises a sterile protective membrane sealingly connected with the circumference of the adapter substrate and extends outward along the circumferential direction to cover at least a portion of the robotic arm.

Note that the above are only exemplary embodiments of the present disclosure and the applied technical principles. Those skilled in the art would appreciate that the present disclosure is not limited to specific embodiments herein, and those skilled in the art could make various apparent changes, readjustments and substitutions without departing from the scope of protection of the present disclosure. Thus, although the present disclosure is described in more detail by the above embodiments, the present disclosure is not limited to the above embodiments. Without departing from the concept of the present disclosure, more other equivalent embodiments may be included, and the scope of the present disclosure is determined by the scope of the appended claims.

## Claims

1. A surgical tool, comprising:
an arm body;
an end instrument arranged at a distal end of the arm body;
at least one sliding block comprising a connecting interface provided on the sliding block, the connecting interface configured to couple with an external device and receive driving from the external device; and
at least one driving wire for driving the arm body and/or the end instrument, wherein a first end of the driving wire is fixedly connected with the sliding block, and the sliding block is configured for pushing and/or pulling the driving wire under the received driving.

2. The surgical tool according to claim 1, further comprising:
a housing located at a proximal end of the arm body and configured for accommodating the at least one sliding block, the housing comprising at least one housing window in which the connecting interface of the at least one sliding block is located.

3. The surgical tool according to claim 2, further comprising:
at least one first deformable membrane sealingly provided on the housing and configured for covering the at least one housing window, wherein the connecting interface is sealingly connected with the first deformable membrane and is configured to receive driving through deformation of the at least one first deformable membrane.

4. The surgical tool according to claim 2 or 3, wherein
the at least one housing window comprises at least one first housing window located on a first side of the housing and at least one second housing window located on a second side of the housing, wherein the at least one sliding block comprises at least one first sliding block and at least one second sliding block, the connecting interface of the at least one first sliding block is located in the at least one first housing window, and the connecting interface of the at least one second sliding block is located in the at least one second housing window.

5. The surgical tool according to claim 4, wherein the at least one housing window further comprises a third housing window located on a third side of the housing, the at least one sliding block further comprises a third sliding block, and the connecting interface of the third sliding block is located in the third housing window.

6. The surgical tool according to claim 4 or 5, wherein
the surgical tool further comprises at least one stop structure provided on the first side and/or the second side of the housing; and/or
the at least one driving wire comprises at least one arm body driving wire, a first end of the arm body driving wire is fixedly connected with the first sliding block and/or the second sliding block, and a second end of the arm body driving wire is directly or indirectly connected with the arm body; and/or
at least one guiding mechanism is provided within the housing, and the at least one sliding block is provided on the at least one guiding mechanism.

7. The surgical tool according to claim 5, wherein
the end instrument comprises an end effector;
the at least one driving wire further comprises at least one effector driving wire, a distal end of the at least one effector driving wire is connected with the end effector, and a proximal end of the at least one effector driving wire is fixedly connected with the at least one third sliding block.

8. The surgical tool according to any one of claims 1 to 7, wherein at least a portion of the arm body is deformable or bendable, one or more arm body driving wires of the at least one driving wire are arranged throughout the arm body, and a push and/or pull motion of the arm body driving wires is configured to drive the deformable portion to deform or to drive the bendable portion to bend.

9. The surgical tool according to any one of claims 2 to 8, further comprising:
a balancing valve provided on the housing; and/or
a handle provided on the housing; and/or
a communication interface provided on the housing.

10. The surgical tool according to any one of claims 3 to 8, wherein
the first deformable membrane comprises a sheet membrane adapted to a shape of the housing, the sheet membrane sealingly provided on an inner surface or outer surface of the housing to cover the at least one housing window; or
the first deformable membrane comprises at least one sheet membrane respectively corresponding to the at least one housing window, the at least one sheet membrane sealingly connected with the housing respectively to cover the at least one housing window.

11. A surgical robot system, comprising:
at least one robotic arm comprising at least one driving device, wherein the at least one driving device comprising at least one driving interface, the at least one driving device configured to drive the at least one driving interface to move;
at least one surgical tool according to any one of claims 1 to 10; and
at least one connecting adapter configured for detachable connection with the robotic arm and the surgical tool, the connecting adapter comprising at least one first interface for coupling with a connecting interface of the surgical tool and at least one second interface for coupling with the driving interface.

12. The surgical robot system according to claim 11, wherein the connecting adapter comprises:
an adapter substrate comprising at least one transmission window, the adapter substrate being detachably connected with the robotic arm and the surgical tool, respectively;
at least one second deformable membrane sealingly provided on the adapter substrate to cover the at least one transmission window;
at least one transmission member sealingly provided on the second deformable membrane and located in the at least one transmission window, the transmission member comprising a first interface located on a first side of the second deformable membrane and a second interface located on a second side of the second deformable membrane, the at least one transmission member configured for driving the connecting interface to linearly move through deformation of the at least one second deformable membrane under linear driving of the driving interface, thereby driving the sliding block to linearly move.

13. The surgical robot system according to claim 12, wherein
the at least one transmission window comprises a first transmission window and a second transmission window,
the at least one transmission comprises at least one first transmission member within the first transmission window and at least one second transmission member within the second transmission window.

14. The surgical robot system according to claim 13, wherein
the first transmission window and the at least one first transmission member are arranged mirror-symmetrically with respect to the second transmission window and the at least one second transmission member.

15. The surgical robot system according to claim 13 or 14, wherein
the adapter substrate comprises a left side substrate, a right side substrate and a middle substrate, and the first transmission window and the second transmission window are respectively provided on the left side substrate and the right side substrate; and/or
the at least one transmission window further comprises a third transmission window provided on the middle substrate.

16. The surgical robot system according to claim 15, wherein the adapter substrate further comprises:
at least one stop portion provided on an inner side of the left side substrate and/or the right side substrate and protruding from the left side substrate and/or the right side substrate; and/or
at least one connecting structure provided on an outer side of the left side substrate and/or the right side substrate; and/or
at least one shielding portion provided on an outer side of the left side substrate and/or the right side substrate and extending outwardly from the substrate surface, the shielding portion configured for shielding a portion of the transmission member exposed on the outer side of the adapter substrate; and/or
at least one communication contact provided on the adapter substrate, the at least one communication contact configured for forming a communication connection between the first side and the second side of the adapter substrate; and/or
at least one ground pin provided on the adapter substrate.

17. The surgical robot system according to any one of claims 11 to 16, wherein the adapter substrate is in a " 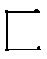 " shape; or
the adapter substrate is provided with at least one folding line thereon, and the adapter substrate is foldable along the folding line to form a " 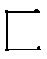 " shape.

18. The surgical robot system according to any one of claims 11 to 17, wherein the at least one connecting adapter further comprises:
at least one sterile protective membrane sealingly connected with a circumference of the at least one adapter substrate and extending outwardly along the circumferential direction to cover the at least one robotic arm.

19. The surgical robot system according to any one of claims 11 to 18, wherein the at least one driving device further comprises:
at least one motor; and
at least one transmission mechanism coupled with the at least one motor, the at least one driving interface fixedly provided on the at least one transmission mechanism, the transmission mechanism configured for converting rotational motion of the motor into linear motion to drive the at least one transmission member to move, to drive the connecting interface of the surgical tool to linearly move through deformation of the at least one second deformable membrane.

20. The surgical robot system according to any one of claims 11 to 19, wherein the driving device further comprises:
a body comprising a receiving slot provided at a distal end and at least one driving window provided on an inner wall of the receiving slot, wherein the at least one driving interface is located within the at least one driving window.
